Europäisches Patentamt

European Patent Office

Office européen des brevets

(11) Veröffentlichungsnummer: **0 295 575 A1**

## (12) EUROPÄISCHE PATENTANMELDUNG

(21) Anmeldenummer: 88109222.5

(22) Anmeldetag: 09.06.88

(51) Int. Cl.⁴: **A61B 5/08** , **G01F 1/40**

(30) Priorität: 16.06.87 BG 80178/87

(43) Veröffentlichungstag der Anmeldung:
21.12.88 Patentblatt 88/51

(84) Benannte Vertragsstaaten:
**CH DE FR GB IT LI NL**

(71) Anmelder: **INSTITUT PO TECHNITSCHESKA KIBERNETIKA I ROBOTIKA**
**Akademik Bontschev-Strasse, Block 2**
**Sofia(BG)**

(72) Erfinder: **Beyazov, Yordan Yordanov**
**131-B, Oborishte Street**
**Sofia(BG)**
Erfinder: **Nenov, Sasho Georgiev**
**4, Zar Shishman Street**
**Sofia(BG)**
Erfinder: **Peychev, Vlayko Slavchev**
**Block 65-E Komplex Mladost-1**
**Sofia(BG)**

(74) Vertreter: **Finck, Dieter et al**
**Patentanwälte v. Füner, Ebbinghaus, Finck**
**Mariahilfplatz 2 & 3**
**D-8000 München 90(DE)**

(54) Durchflussumformer, insbesondere für die Diagnostik von Lungenerkrankungen.

(57) Der Durchflußumformer, der insbesondere für die Diagnostik von Lungenerkrankungen verwendet wird, hat einen Ringkörper (1) und ein in ihm angeordnetes zylindrisches Rohr (2). In dem Ringkörper (1) ein Heizelement (12) angeordnet. Der Ringkörper (2) hat innenseitig wenigstens zwei im Abstand vorgesehene Umfangskanäle (8, 9), von denen jeder mit einem am Ringkörper (1) sitzenden Meßstutzen (10, 11) verbunden ist. An dem zylindrischen Rohr (2) ist einlaß- und auslaßseitig jeweils ein Diffusor (3 bzw. 5) angeschlossen. In dem zylindrischen Rohr (2) ist austauschbar ein zylindrischer Einsatz (14) angeordnet, in dessen Außenfläche axiale Längskanäle (15) mit einem eine laminare Strömung verursachenden Querschnitt ausgebildet sind. Die axialen Längskanäle (15) sind über die Umfangskanäle (8, 9) mit den Meßstutzen (10, 11) verbunden. Durch den zylindrischen Einsatz (14) erstreckt sich wenigstens ein axialer, einlaß- und auslaßseitig mit Abrundungen (17) versehener Durchflußkanal (16), dessen Querschnitt wenigstens das Zweifache der Querschnitte der axialen Längskanäle (15) beträgt.Dem zylindrischen Einsatz (14) ist ein am auslaßseitigen Diffusor (5) anbringbarer, mit Öffnungen (7) versehener Deckel (6) zugeordnet, deren Durchflußquerschnitt bevorzugt gleich oder kleiner ist als der Querschnitt des oder der axialen Durchflußkanäle (16) im Einsatz (14).

FIG.1

## DURCHFLUSSUMFORMER, INSBESONDERE FÜR DIE DIAGNOSTIK VON LUNGENERKRANKUNGEN

Die Erfindung betrifft einen Durchflußumformer, inbesondere für die Diagnostik von Lungenerkrankungen, mit einem Ringkörper, in welchem ein Heizelement angeordnet ist und der innenseitig wenigstens zwei im Abstand vorgesehene Umfangskanäle aufweist, von denen jeder mit einem am Ringkörper sitzenden Meßstutzen verbunden ist, und mit einem in dem Ringkörper angeordneten zylindrischen Rohr, an das einlaß- und auslaßseitig jeweils en Diffusor angeschlossen ist, wobei axiale Längskanäle ausgebildet sind, welche über die Umfangskanäle mit den Meßstutzen in Verbindung stehen.

Bei einem solchen, aus dem Prospekt der niederländischen Firma Gould bekannten Durchflußumformer sind mehrere tausend axiale Längskanäle vorgesehen, die einen dreieckförmigen Querschnitt haben und den ganzen Querschnitt des Rohres besetzen. Um Untersuchungen im gesamten Bereich wirksam durchführen zu können, ist ein Satz von zehn Durchflußumformern mit verschiedenen Parametern und Aussenabmessungen erforderlich.

Die Herstellung eines jeden Durchflußumformers ist relativ kompliziert, so daß die Bereitstellungen eines vollen Satzes aus Durchflußumformern zur Abdeckung des gesamten Untersuchungsbereichs sehr aufwendig ist.

Die der Erfindung zugrunde liegende Aufgabe besteht deshalb darin, den gattungsgemäßen Durchlußumformer so auszubilden, daß zur Abdeckung des gesamten Untersuchungsbereichs ein und derselbe Durchflußumformer verwendet werden kann, wobei für die verschiedenen Untersuchungsbereiche maximal zwei Elemente auf einfache Weise ausgetauscht zu erden brauchen.

Diese Aufgabe wird ausgehend von dem Durchflußumformer der gattungsgemäßen Art dadurch gelöst, daß in dem zylindrischen Rohr austauschbar ein zylindrischer Einsatz angeordnet ist, in dessen Außenfläche die axialen Längskanäle mit einem eine laminare Strömung verursachenden Querschnitt ausgebildet sind, und durch den sich wenigstens ein axialer einlaß- und auslaßseitig mit Abrundungen versehener Durchflußkanal erstreckt, dessen Querschnitt wenigstens das Zweifache der Querschnitte der axialen Längskanäle beträgt, und daß jedem Einsatz ein am auslaßseitigen Diffusor anbringbarer, mit Öffnungen versehener Deckel zugeordnet ist.

Zweckmäßigerweise ist der Durchflußquerschnitt der Öffnungen im Deckel gleich oder kleiner als der Querschnitt des oder der axialen Durchflußkanäle im Einsatz.

Während in dem oder den axialen Durchflußkanälen im Einsatz die Strömung turbulent ist, ist in den axialen Längskanälen die Reynoldszahl der Strömung kleiner als der kritische Wert, d.h. die Strömung ist laminar, so daß die Abhängigkeit zwischen dem Druckabfall zwischen den Meßstutzen und dem Druchfluß unabhängig von dem turbulenten Charakter der Strömung in dem oder den axialen Durchflußkanälen linear ist. Dadurch ist bei Erzielung einer hohen Meßgenauigkeit anstelle eines Satzes nur ein einziger Umformer zur Abdeckung des gesamten Untersuchungsbereiches mit gleichbleibenden Außenabmessungen erforderlich, bei welchem lediglich die Einsätze und die Deckel mit den Öffnungen dem Untersuchungsbereich entsprechend ausgewechselt zu werden brauchen.

Anhand von Zeichnungen werden Ausführungsbeispiele der Erfindung näher erläutert. Es zeigt:

Fig. 1 im Axialschnitt eine erste Ausführungsform eines Durchflußumformers,

Fig. 2 im Axialschnitt eine zweite Ausführungsform eines Durchflußumformers und

Fig. 3 den Schnitt A-A von Fig. 2.

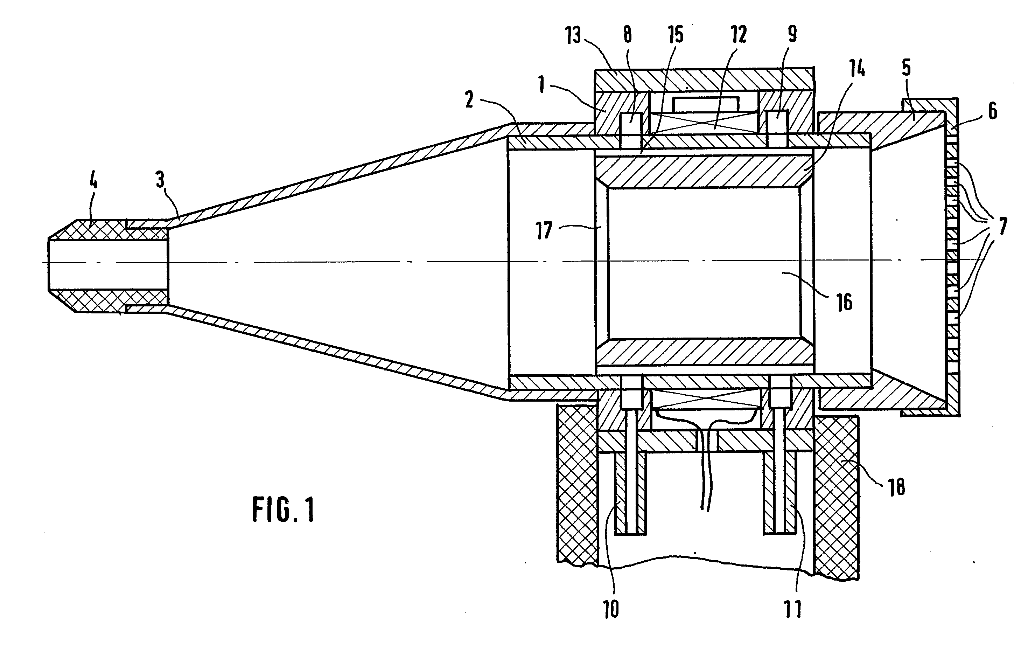

Der in den Fig. 1 bis 3 gezeigte Durchflußumformer hat einen Ringkörper 1, in welchem ein zylindrisches Rohr 2 angeordnet ist, an das sich an einem Ende ein einlaßseitiger Diffusor 3 anschließt, der an seinem anderen Ende ein Mundstück 4 aufweist. An dem zylindrischen Rohr 2 ist auslaßseitig ein weiterer Diffusor 5 angebracht, an dem ein Deckel 6 mit Öffnungen 7 auswechselbar festlegbar ist.

An der Innenfläche des zylindrischen Rohres 2 sind im Abstand zwei Umfangskanäle 8 und 9 ausgebildet, von denen jeder mit einem Meßstutzen 10 bzw. 11 verbunden ist, der am Körper 1 sitzt. Im Körper 1 ist ferner ein Heizelement 12 angeordnet. Der Körper 1 ist umfangsseitig mit einem Mantel 13 abgeschlossen.

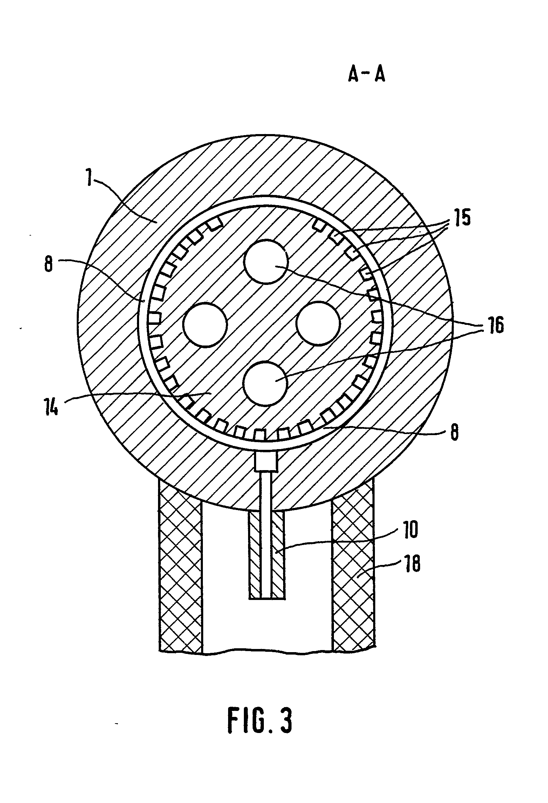

In dem zylindrischen Rohr 2 ist auswechselbar ein zylindrischer Einsatz 14 angeordnet. Der zylindrische Einsatz 14 hat auf seiner Außenfläche axiale Längskanäle 15, wie dies aus Fig. 3 zu ersehen ist. Die axialen Längskanäle 15 sind im Querschnitt rechteckig. Ihre Querschnitte sind gleich groß. In dem zylindrischen Einsatz 14 ist ferner bei der Ausführungsform von Fig. 1 ein axialer Durchflußkanal 16 mit kreisförmigem Querschnitt vorgesehen. Bei dem gezeigten Ausführungsbeispiel hat der Durchflußkanal 16 einen Querschnitt, der zehnmal größer ist als di Summe der Querschnitte der axialen Längskanäle 15. Der axiale Durchflußkanal 16 hat einlaß- und ausflaßseitig jeweils eine Abrundung 17 zur Verringerung des Strömungswiderstandes. Anstelle der zylindrischen Form kann der axiale Durchflußkanal 16 auch eine Kombination aus einem Konfusor und Diffusor mit oder ohne dazwischenliegenden zylindrischen Abschnitt bestehen.

An dem Ringkörper 1 ist ein Griff 18 befestigt. Der Körper 1 und das zylindrische Rohr 2 können auch aus einem Stück gefertigt werden.

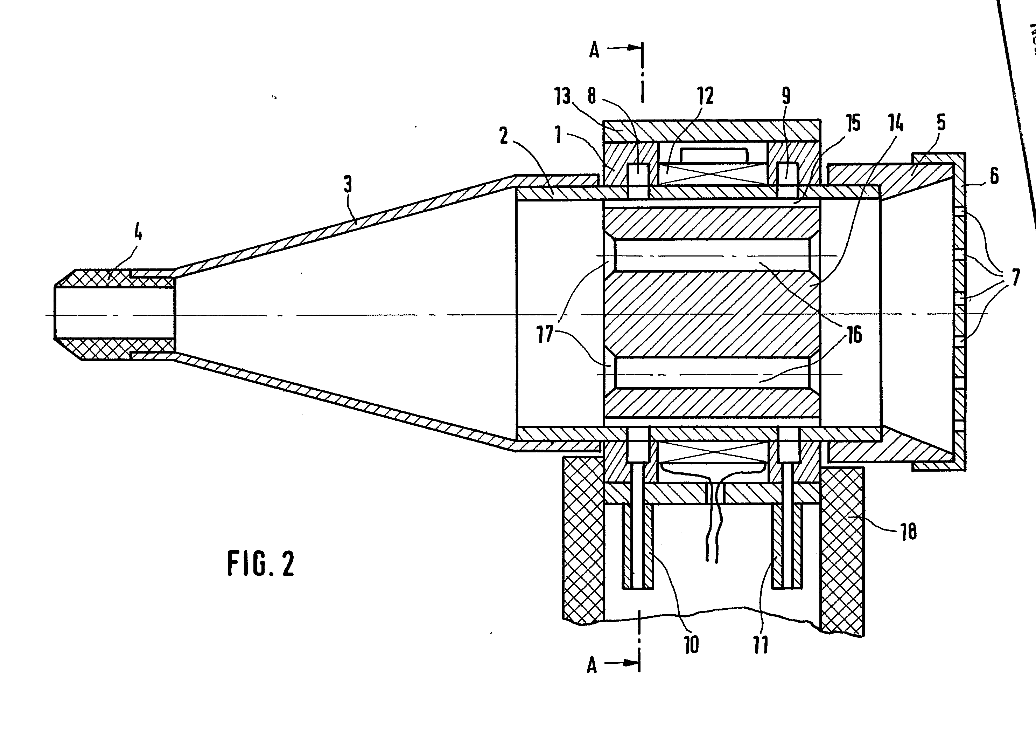

Die Zahl der eine laminare Strömung gewährleistenden axialen Längskanäle 15 ist für jeden zylindrischen Einsatz 14 konstant, während zur Abdeckung des Untersuchungsbereiches die Anzahl und der Durchmesser des oder der axialen Durchlaßkanäle 16 entsprechend ausgewählt sind, wovon ein Beispiel in Fig. 2 und 3 gezeigt ist. Die Strömung in den axialen Durchlaßkanälen 16 ist turbulent. Der gesamte Durchflußquerschnitt der Öffnungen 7 eines jeden austauschbaren Deckels 6 ist gleich oder kleiner dem gesamten Durchflußquerschnitt der axialen Durchflußkanäle 16 des entsprechenden austauschbaren zylindrischen Einsatzes 14.

Die Meßstutzen 10 und 11 können in Reihe mit einem pneumoelektrischen Umformer für den Differenzdruck, mit einer elektronischen Schaltung für die Abstimmung, mit einem Mikroprozessorsystem für die Datenverarbeitung und einem Anzeige- bzw. Registriergerät für das Untersuchungsergebnis verbunden werden.

Der Durchflußumformer arbeitet folgendermaßen: Der durch das Mundstück 4 und den einlaßseitigen Diffusor 3 eintretende Strom wird in zwei Teilströme aufgeteilt. Der eine wesentlich kleinere Teilstrom geht durch die axialen Längskanäle 15 jeweils als laminarer Strom, während der zweite Teilstrom als Hauptstrom durch den oder die axialen Durchflußkanäle 16 in turbulenter Strömung hindurchfließt. Infolge des laminaren Charakters der Strömung durch die axialen Längskanäle 15 ist die Abhängigkeit zwischen dem an den Meßstutzen 10 und 11 vorhandenen Druckabfalls und dem Durchfluß linear, und zwar unabhängig von dem turbulenten Charakter der Strömung durch den oder die axialen Durchflußkanäle 16. Die Abrundungen 17 erhöhen den Durchflußkoeffizienten erheblich. Der austauschbare Deckel 6 verhindert eine Rückströmung, die zu Messfehlern führen könnte.

## Ansprüche

1. Durchflußumformer, insbesondere für die Diagnostik von Lungenerkrankungen, mit einem Ringkörper (1), in welchem ein Heizelement (12) angeordnet ist und der innenseitig wenigstens zwei im Abstand vorgesehene Umfangskanäle (8, 9) aufweist, von denen jeder mit einem am Ringkörper (1) sitzenden Meßstutzen (10, 11) verbunden ist, und mit einem in dem Ringkörper (1) angeordneten zylindrischen Rohr (2), an das einlaß-und auslaßseitig jeweils ein Diffusor (3 bzw. 5) angeschlossen ist, wobei axiale Längskanäle (15) ausgebildet sind, welche über die Umfangskanäle (8, 9) mit dem Meßstutzen (10, 11) in Verbindung stehen, dadurch **gekennzeichnet,** daß in dem zylindrischen Rohr (2) austauschbar ein zylindrischer Einsatz (14) angeordnet ist, in dessen Außenfläche die axialen Längskanäle (15) mit einem eine laminare Strömung verursachenden Querschnitt ausgebildet sind, und durch den sich wenigstens ein axialer einlaß- und auslaßseitig mit Abrundungen (17) versehener Durchflußkanal (16) erstreckt, dessen Querschnitt wenigstens das Zweifache aller Querschnitte der axialen Längskanäle (15) beträgt, und daß jedem Einsatz (14) ein am auslaßseitigen Diffusor (5) anbringbarer, mit Öffnungen (7) versehener Deckel (6) zugeordnet ist.

2. Durchflußumformer nach Anspruch 1, dadurch **gekennzeichnet,** daß der Durchflußquerschnitt der Öffnungen (7) im Deckel (6) gleich oder kleiner ist als der Querschnitt des oder der axialen Durchflußkanäle (16) im Einsatz (14).

FIG.1

EP 0 295 575 A1

FIG. 2

A- A

FIG. 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | HEWLETT PACKARD JOURNAL, vol. 30, no. 9, september 1979, pages 20-24, Palo Alto, US; M.R. BLAIS et al.: "Automated pulmonary function measurements" * Seite 22, rechte Spalte, Zeile 11 - Seite 23, linke Spalte, Zeile 29; Figur 4 * --- | 1 | A 61 B    5/08 G 01 F    1/40 |
| A | DE-A-2 907 788  (THE PERKIN-ELMER CORP.) * Seite 9, Zeilen 6-18; Figur * --- | 1 | |
| A | DE-A-2 030 775  (SIEMENS AG) * Seite 1, Kurzauszug, Zeilen 6-12; Figur 2 * ----- | 1 | |

**RECHERCHIERTE SACHGEBIETE (Int. Cl.4)**

A 61 B    5/00
G 01 F    1/00

Der vorliegende Recherchenbericht wurde für alle Patentansprüche erstellt

| Recherchenort | Abschlußdatum der Recherche | Prüfer |
|---|---|---|
| BERLIN | 01-09-1988 | WEIHS J.A. |

EPO FORM 1503 03.82 (P0403)